(19) Europäisches Patentamt
European Patent Office
Office européen des brevets

(11) Veröffentlichungsnummer: **0 004 260**
**B1**

(12) # EUROPÄISCHE PATENTSCHRIFT

(21) Anmeldenummer: **78101241.4**

(22) Anmeldetag: **27.10.78**

(51) Int. Cl.³: **C 07 D 471/04,**
C 07 D 455/02,
A 61 K 31/445,//
(C 07 D 471/04 209/00,
221/00)

(54) **Substituierte Chinolizidin- und Indolizidinmethanolderivate, Verfahren zur Herstellung derselben und Arzneimittel, welche diese enthalten**

(30) Priorität: **22.03.78 JP 31652/78**
**22.03.78 JP 31653/78**
**23.05.78 JP 60654/78**
**23.05.78 JP 60656/78**

(43) Veröffentlichungstag der Anmeldung:
**03.10.79 Patentblatt 79/20**

(45) Bekanntmachung des Hinweises auf die Patenterteilung:
**29.10.80 Patentblatt 80/22**

(84) Benannte Vertragsstaaten:
**BE CH FR GB SE**

(56) Entgegenhaltungen:
**DE - A - 1 545 661**
**CHEMISCHE BERICHTE, 90. Jahrgang, 1957, Weinheim, Bergstraße**
**K. WINTERFELD et al. "Synthese der Chinolizidin-Carbonsäure-(1) (DL-1-Lupininsäure)", Seiten 863-867**

(73) Patentinhaber: **Hokuriku Pharmaceutical Co., Ltd**
**1-Chome, 3 - 14 Tatekawacho Katsuyamashi**
**Fukui (JP)**

(72) Erfinder: **Koshinaka, Eiichi**
**2-6-3 Asahicho Katsuyamashi**
**Fukui (JP)**
**Ogawa, Nobuo**
**4-2-20 Honmachi Katsuyamashi**
**Fukui (JP)**
**Kurata, Sakae**
**1-6-56 Asahicho Katsuyamashi**
**Fukui (JP)**
**Jamagischi, Kagari**
**2-5-44 Asahicho Katsuyamashi**
**Fukui (JP)**
**Kato, Hideo**
**1-11-27 Motomachi Katsuyamashi**
**Fukui (JP)**
**Matsubara, Issei**
**11-31 Hojioka Eiheijicho Yoshidagun**
**Fukui (JP)**

(74) Vertreter: **Eitle, Werner, Dipl.-Ing. et al**
**Arabellastrasse 4**
**D - 8000 München 81 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Courier Press, Leamington Spa, England.

Substituierte Chinolizidin- und Indolizidinmethanol-derivate, Verfahren zur Herstellung derselben und Arzneimittel, welche diese enthalten

Die Herstellung von Diphenyl-chinolizidyl-(1)-carbinol wird in Chem. Ber. *90*, 863 bis 867 (1957) bereits beschrieben, ohne auf die Anwendung der Verbindungen hingewiesen wird.

Aufgabe der Erfindung ist es, ein Arzneimittel zur Verfügung zu stellen, das antihistaminische, analgetische und spasmolytische Wirkungen aufweist.

Eine weitere Aufgabe der Erfindung ist es, Zwischenprodukte zur Verfügung zu stellen, aus denen die erwähnten substituierten Chinolizidin- und Indolizidinderivate in einer einfachen Arbeitsweise und mit hoher Ausbeute hergestellt werden können.

Gegenstand der Erfindung sing $\alpha,\alpha$-disubstituierte Chinolizidin- und Indolizidinmethanolderivate der Formel (I)

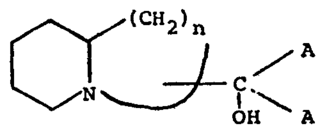

(I)

worin A die Phenyl- oder 2-Thienylgruppe und n eine ganze Zahl von 3 oder 4 bedeutet, wobei, wenn A die Phenylgruppe und n eine Zahl von 4 ist, der $\alpha,\alpha$-Diphenylhydroxymethylrest in 2- oder 3-Stellung des Chinolizidinkerns gebunden ist, sowie ihre Säureadditionssalze und quaternären Salze.

Die Verbindungen der Formel (I) weisen antihistaminische, antiemetische und anticholinergische Wirkungen auf und können gleichzeitig als Zwischenprodukt zur Herstellung der oben genannten substituierten Chinolizidin- und Indolizidinderivate verwendet werden.

Ausserdem weisen die quaternären Salze der Formel (VI)

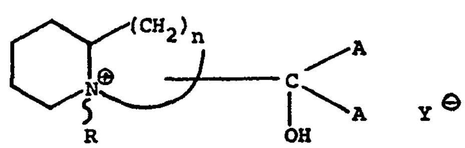

(VI)

worin n und A die oben angegebenen Bedeutungen haben und R eine niedrige Alkylgruppe und Y einen Säurerest bedeutet, Arzneimittelwirkung, nämlich anticholinergische Aktivatät auf und sind als Spasmolytica oder Antigeschwürmittel geeignet.

Die Erfindung betrifft auch ein Verfahren zur Herstellung der Derivate der Formel (I) und ihrer Salze, das dadurch gekennzeichnet ist, dass eine Verbindung der Formeln (II) oder (III):

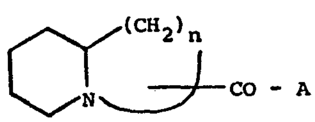

(II)

oder

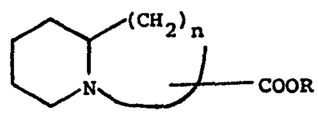

(III)

worin n und A sowie R die oben angegebenen Bedeutungen haben, mit einer Metallverbindung der Formeln (IV) oder (V):

A — MgX (IV)

oder

A — Li (V)

wobei A die oben angegebene Bedeutung hat und X ein Halogenatom bedeutet, umgesetzt wird und gegebenenfalls die erhaltene Verbindung mit einer pharmazeutisch verträglichen anorganischen oder organischen Säure oder mit einem Quaternisierungsmittel umgesetzt wird.

Die Umsetzung der Verbindung der Formel (II) oder (III) mit der Metallverbindung der Formel (IV) oder (V) wird bei Raumtemperatur oder durch Erhitzen unter Rückfluss in einem organischen Lösungsmittel durchgeführt. Als Lösungsmittel sing z.B. trockener Äther oder Tetrahydrofuran bevorzugt. Wenn die Verbindung der Formel (II) eingesetzt wird, wird die Metallverbindung der Formel (IV) oder (V) in einer Menge von einem Mol oder mehr eingesetzt.

Andererseits, wenn die Verbindung der Formel (III) eingesetzt wird, sollte die Metallverbindung der Formel (IV) oder (V) in einer Menge von 2 Molen oder mehr verwendet werden.

Die Ausgangsmaterialien der Formeln (II) und (III) enthalten sterische Isomeren und können als Gemisch von Diastereoisomeren oder als reine, isolierte Isomere verwendet werden. Die Verbindungen der Formel (I) können jeweils als Gemisch oder reine Isomere erhalten werden.

Die reinen Isomeren können durch fraktionierte Kristallisation des Diastereoisomerengemisches oder durch Säulenchromatografie gewonnen werden.

Die Verbindungen der Formeln (I) und (VI) können mit einer pharmazeutisch verträglichen, anorganischen Säure, z.B. Salzsäure, Bromwasserstoffsäure, Schwefelsäure usw. oder organischen Säuren. z.B. Maleinsäure, Fumarsäure, Zitronensäure, Weinsäure, Oxalsäure, Bernsteinsäure usw. in ein entsprechendes Säureadditionssalz überführt werden.

Die quaternären Salze der Formel (VI) können mit den Verbindungen der Formel (VII)

$$R—Y \qquad (VII)$$

worin R und Y die oben angegebenen Bedeutungen haben, hergestellt werden.

Als Alkylgruppe für R kommen Methyl, Äthyl, Propyl, Butyl usw. in Betracht. Als Säurerest kommen Chlor-, Brom- oder Jodatome, der Schwefelsäurerest, der Alkylsulfatrest und dergleichen in Frage.

Die Quarternisierung kann in An- oder Abwesenheit eines Lösungsmittels vorgenommen werden. Als Lösungsmittel werden z.B. Äther, Aceton und Alkohol, wie Methanol oder Äthanol, venwendet. Die Umsetzung wird bei einer Temperatur zwischen 5°C bis 100°C, vorzugsweise 10 bis 40°C, gegebenenfalls in einem abgeschmolzenen Rohr, vorgenommen.

Die quaternären Salze der Formel (VI) enthalten auch die Stereoisomeren (Trans- und Cisisomer), die als Gemisch oder reine Isomere nach Umkristallisation erhalten werden können.

Die Ausgangsmaterialien der Formeln (II) und (III) sind teilweise bekannt, teilweise neu. Die neuen Ausgangsmaterialien können z.B. wie folgt hergestellt werden:

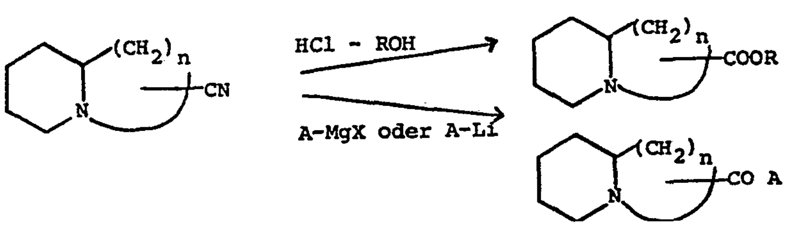


Die erfindungsgemässen Verbindungen, besonders der quaternären Salze, weisen im Vergleich zu Atropin lediglich verminderte Nebenwirkungen, wie Durst, Pupillenerweiterung usw. auf und können daher praktisch als Spasmolytica und Antigeschwürmittel verwendet werden.

Bei der klinischen Anwendung können die Verbindungen in einer Dosis von 1 mg bis 100 mg, bevorzugt 3 bis 30 mg, dreimal am Tag per os verabreicht oder als Injektion in einer entsphrechenden Menge verwendet werden. Die Arzneimittel welche die Verbindungen gemäss der Erfindung enthalten, können die üblichen Verdünnungsmittel und Träger enthalten.

Im forgenden wird die Erfindung anhand der Ausführungsbeispiele näher erläutert.

Beispiel 1

*3-[1,1-(Dithien-2-yl)-1-hydroxymethyl]-(a)-trans-chinolizidin*

Eine Lösung von 0,34 g 3-(Thenoyl)(a)-transchinolizidin in trockenem Äther wird tropfenweise zu einer Lösung von Thienylmagnesiumbromid (hergestellt aus 0,5 g Magnesium und 0,35 g 2-Bromthiophen) in trockenem Äther unter Kühlung zugesetzt und dann zum Rüchfluss drei Stunden lang erhitzt. Nach Beendigung der Umsetzung wird Wasser zum Umsetzungsgemisch zugesetzt. Die Ätherphase wird abgetrennt und mit 10%-iger wässriger Salzsäure extrahiert. Die wässrige Phase wird durch Zusetzen einer Natriumhydroxidlösung alkalisch gemacht und dann mit Chloroform extrahiert. Die erhaltene Chloroformphase wird mit Wasser gewaschen und getrocknet. Nach Abdestillieren des Lösungsmittels wird der Rückstand aus Isopropyläther umkristallisiert, so dass 0,55 g farblose Nadeln mit einem Schmelzpunkt von 147 bis 148°C erhalten werden.

Elementaranalyse für $C_{18}H_{23}NOS_2$

| | | | |
|---|---|---|---|
| Berechnet: | C 64,82 | H 6,95 | N 4,20 |
| Gefunden: | 64,91 | 7,04 | 3,96 |

Beispiel 2

*2-[1,1-(Dithien-2-yl)-1-hydroxymethyl]-(e)-trans-chinolizidin*

Eine Lösung von 3,80 g 2-Äthoxycarbonyl-(e)-trans-chinolizidin in trockenem Tetrahydrofuran wird zu einer Lösung von Thienylmagnesiumbromid (hergestellt aus 1,35 g Magnesium und 8,80 g 2-Bromthiophen) in trockenem Tetrahydrofuran zugestezt und das erhaltene Gemisch wird gerührt. Nach Beenden der Umsetzung wird das Gemisch mit einer Lösung von Natriumhydroxid versetzt und filtriert. Das erhaltene Filtrat wird eingeengt und mit Wasser und Äther versetzt. Die ätherische Phase wird abgetrennt und mit 5%-iger wässriger Salzsäure extrahiert.

Nachdem die wässrige Phase mit einer Natriumhydroxidlösung alkalisch gemacht wurde, wird sie mit Äther extrahiert. Die ätherische Phase wird mit Wasser gewaschen und getrocknet. Nach Abdestillieren des Lösungsmittels wird der Rückstand aus Benzol/Isopropyläther umkristallisiert, so dass 4,64 g farblose Prismen mit einem Schmelzpunkt von 149 bis 150°C erhalten werden.

Elementaranalyse für $C_{18}H_{23}NOS_2$

| | | | |
|---|---|---|---|
| Berechnet: | C 64,82 | H 6,95 | N 4,20 |
| Gefunden: | 64,99 | 6,96 | 3,85 |

Beispiel 3

*1-[1,1-(Dithien-2-yl)-1-hydroxymethyl]-(a)-trans-chinolizidin-Methylbromid*

Eine Lösung von 0,05 g 1-[1,1-(Dithien-2-yl)-1-hydroxymethyl]-(a)-trans-chinolizidin in 2 ml Aceton wird zu 1,0 ml Methylbromid zugesetzt und bei Raumtemperatur 60 Stunden lang gerührt. Die auskristallisierten Kristalle werden filtriert und aus Aceton/Methanol umkristallisiert, so dass 0,03 g farblose Prismen mit einem Schmelzpunkt von 167 bis 170°C (Zersetzung) erhalten werden.

Elementaranalyse für $C_{19}H_{26}BrNOS_2$

| | | | |
|---|---|---|---|
| Berechnet: | C 53,26 | H 6.12 | N 3,27 |
| Gefunden: | 52,96 | 6,15 | 3,21 |

Beispiel 4

*2-[1,1-Diphenyl-1-hydroxymethyl]-(e)-trans-chinolizidin*

Eine Lösung von 5,3 g 2-Benzoyl-(e)-chinolizidin in trokkenem Äther wird tropfenweise zu einer Lösung von Phenyllithium (hergestellt aus 0,46 g Lithium und 5,2 g Brombenzol) in trockenem Äther zugesetzt und dann 15 Minuten lang zum Rüchfluss erhitzt. Nach der Beendigung der Umsetzung wird das Umsetzungsgemisch mit Wasser versetzt und der Äther abdestilliert. Der Rückstand wird zu Hexan zugesetzt. Die abgeschiedenen Kristalle werden abfiltriert und mit Wasser und dann mit n-Hexan gewashcen. Nach dem Trocknen entstehen 4,76 g farblose Kristalle. Durch Umkristallisierung aus Isopropanol erhält man farblose plattenförmige Kristalle mit einem Schmelzpunkt von 188 bis 189°C.

IR (KBr): 3400 $cm^{-1}$ (OH)

Elementaranalyse für $C_{22}H_{27}NO \cdot 1/4\ H_2O$

| | | | |
|---|---|---|---|
| Berechnet: | C 81,06 | H 8,50 | N 4,30 |
| Gefunden: | 81,11 | 8,63 | 4,30 |

Beispiel 5

*2-[1,1-Diphenyl-1-hydroxymethyl]-(e)-trans-chinolizidin*

Eine Lösung von 5,3 g 2-Äthoxycarbonyl-(e)-trans-chinolizidin in trockenem Äther wird tropfenweise zu einer Lösung von Phenyllithium (hergestellt aus 0,39 g Lithium und 4,79 g Brombenzol) in trockenem Äther zugesetzt. Nachdem das erhaltene Gemisch 30 Minuten lang unter Rückfluss gehalten und wie in Beispiel 4 angegeben behandelt wurde, entstehen 2,77 g farblose Kristalle. Durch Umkristallisation aus Isopropanol erhält man farblose plattenförmige Kristalle mit einem Schmelzpunkt von 188 bis 189°C. die erhaltene Verbindung entspricht dem Produkt in Beispiel 4 in IR und TLC und zeigt keine Senkung des Schmelzpunktes bei der Mischprobe.

Beispiel 6

*rel-(2 S, 8aR)-2-[1,1-Dithien-2-yl)-1-hydroxymethyl]-indolizidin*

Eine Lösung von 0,37 g rel-(2 S, 8aR)-2-Äthoxycarbonylindolizidin in trockenem Äther wird tropfenweise zu einer Lösung von Thienylmagnesiumbromid (hergestellt aus 0,14 g Magnesium und 1,8 g 2-Bromthiophen) in trockenen Äther unter Kühlung zugesetzt und bei Raumtemperatur 10

Minuten lang gerührt. Nach Beendigung der Umsetzung wird das Umsetzungsgemisch mit Wasser versetzt und mit Äther extrahiert. Die ätherische Phase wird mit Wasser gewaschen und getrocknet. Durch Abdestillieren des Lösungsmittels und Umkristallisieren des Rückstandes aus Isopropyläther erhält man 0,10 g farblose Nadelkristalle mit einem Schmelzpunkt von 130 bis 131°C.

IR ($CHCl_3$): ca. 3200 $cm^{-1}$ (OH)

Elementaranalyse für $C_{17}H_{21}NOS_2$

| | | | |
|---|---|---|---|
| Berechnet: | C 63,91 | H 6,63 | N 4,38 |
| Gefunden: | 63,61 | 6,65 | 4,13 |

Beispiel 7

*rel-(2R,8aR)-2-(1,1-Diphenyl-1-hydroxymethyl)indolizidin (A) und rel-(2S, 8aR)-2-(1,1-Diphenyl-1-hydroxymethyl)-indolizidin (B)*

2-Benzoylindolizidin wird zu einer Lösung von Phenyllithium (hergestellt aus 1,09 g Metallithium und 12,3 g Brombenzol) in trockenem Äther zugesetzt und 20 Minuten lang unter Rückfluss erhitzt. Nach Beendigung der Umsetzung wird das Umsetzungsgemisch mit Wasser versetzt und mit Äther extrahiert. Die ätherische Phase wird mit Wasser gewaschen und getrocknet. Das Lösungsmittel wird abdestilliert und der Rückstand wird mit n-Hexan gemischt. Die enstandenen Kristalle werden abgesaugt, so dass 11,75 g farblose Kirstalle erhalten werden. Durch Umkristallisierung aus Isopropyläther erhält man farblose Kristalle (A) mit einem Schmelzpunkt von 136 bis 138°C.

IR ($CHCl_3$): 3600 $cm^{-1}$ (OH)

Elementaranalyse für $C_{21}H_{25}NO$

| | | | |
|---|---|---|---|
| Berechnet: | C 82,04 | H 8,20 | N 4,56 |
| Gefunden: | 81,91 | 8,23 | 4,44 |

Die nach der Umkristallisierung erhaltene Mutterlauge wird unter vermindertem Druck getrocknet. Durch Umkristallisieren des Rückstandes aus Isopropyläther erhält man farblose Nadeln (B) mit einem Schmelzpunkt von 132 bis 134°C.

IR ($CHCl_3$): ca. 3200 $cm^{-1}$ (OH)

Elementaranalyse für $C_{21}H_{25}NO$

| | | | |
|---|---|---|---|
| Berechnet: | C 82,04 | H 8,20 | N 4,56 |
| Gefunden: | 81,87 | 8,06 | 4,33 |

Beispiel 8

*1-(1,1-Diphenyl-1-hydroxymethyl)-indolizidin*

Eine Lösung von 2,40 g 1-Äthoxycarbonylindolizidin in 20 ml trockenem Äther wird tropfenweise zu einer Lösung von Phenyllithium (hergestellt aus 0,51 g Metallithium und 6,32 g Brombenzol und 50 ml trockenem Äther) unter Kühlung mit Eis zugesetzt. Nach Rückfluss von ca. 10 Minuten wird Wasser tropfenweise zum Umsetzungsgemisch zugesetzt. Das Gemisch wird mit Äther extrahiert. Die ätherische Phase wird mit einer verdünnten Lösung der Salzsäure extrahiert. Die wässrige Phase wird mit einer Natriumhydroxidlösung alkalisch gemacht und dann mit Äther extrahiert. Die ätherische Phase wird mit Wasser gewaschen und getrocknet. Nach Abdestillieren des Lösungsmittels erhält man 3,58 g gelbliche viskose Substanz, welche durch Umkristallisation aus Isopropyläther farblose Nadelkristalle mit einem Schmelzpunkt von 120 bis 121°C darstellen.

Elementaranalyse für $C_{21}H_{25}NO$

| | | | |
|---|---|---|---|
| Berechnet: | C 82,04 | H 8,20 | N 4,56 |
| Gefunden: | 81,92 | 8,29 | 4,40 |

Massenspektrografie: $C_{21}H_{25}NO$

m/e: 307 ($M^+$), 230, 123 (Grundspitze)

Das erhaltene Produkt ist ein Gemisch von zwei Diastereoisomeren. Die unten angegebenen Verbindungen werden gemäss einer in den obigen Beispielen 1 bis 8 angegebenen Arbeitsweise hergestellt:

| *Verbindung* | *Physikalische Daten* |
|---|---|
| ( 9) 2-[1,1-(Dithien-2-yl-1-hydroxymethyl]-(e)-trans-chinolizidin-methylbromid | Schmelzpunkt 253—255°C (Aceton/Methanol) |
| (10) 3-[1,1-(Dithien-2-yl)-1-hydroxymethyl]-(a)-trans-chinolizidin-methylbromid | Schmelzpunkt 272—273°C (Zers. Äthanol) |
| (11) 3-[1,1-(Dithien-2-yl)-1-hydroxymethyl]-(e)-trans-chinolizidin-methylbromid | Schmelzpunkt 286—288°C (Zers. Aceton/Methanol) |
| (12) 1-[1,1-(Dithien-2-yl)-1-hydroxymethyl]-(a)-trans-chinolizidin | Schmelzpunkt 186—187°C (Isopropyläther) |
| (13) 3-[1,1-(Dithien-2-yl)-1-hydroxymethyl]-(e)-trans-chinolizidin | Schmelzpunkt 176—177°C (Isopropanol/Isopropyläther) |
| (14) 3-[1,1-Diphenyl-1-hydroxy-methyl]-(a)-trans-chinolizidin | Schmelzpunkt 188—189,5°C (Isopropanol) IR($CHCl_3$): 3000 $cm^{-1}$ (OH) |
| (15) 3-[1,1-Diphenyl-1-hydroxy-methyl] (e)-trans-chinolizidin | Schmelzpunkt 166—167°C (Isopropanol) IR($CHCl_3$): 3600 $cm^{-1}$ (OH) |
| (16) rel-(2R, 8aR)-2-[1,1-(Dithien-2-yl)-1-hydroxymethyl]-indolizidin | Schmelzpunkt 101—102°C IR($CHCl_3$): 3590 $cm^{-1}$(OH) |
| (17) 1-[1,1-(Dithien-2-yl)-1-hydroxymethyl]-indolizidin | Schmelzpunkt 139—140°C Isopropyläther (Gemisch der zwei Isomeren) |
| (18) 3-(1,1-Diphenyl-1-hydroxy-methyl)-(a)-trans-chinolizidin-methylbromid | Schmelzpunkt > 300°C (Methanol/Äther) |
| (19) 3-(1,1-Diphenyl-1-hydroxy-methyl)-(e)-trans-chinolizidin-methylbromid | Schmelzpunkt > 300°C (Methanol/Äther) |

Die antiacetylcholinergische Wirkung der erfindungsgemässen quaternären Salze wird in der folgenden Tabelle im Vergleich zu vorbekannten Verbindungen angegeben:

| *VERBINDUNG* | Index *ANTROPIN* = 1 |
|---|---|
| Nr. 10 | 0,39 |
| Nr. 11 | 0,12 |
| Nr. 18 | 0,20 |
| Diphemanil-Methylsulfat | 0,11 |
| Timepidiumbromid | 0,15 |

**Patentansprüche**

1. $\alpha,\alpha$-disubstituierte Chinolizidin- und Indolizidinmethanolderivate der Formel (I)

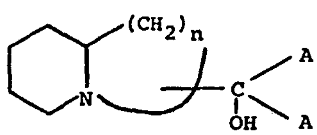
(I)

worin A die Phenyl- oder 2-Thienylgruppe bedeutet und n eine ganze Zahl von 3 oder 4 ist, wobei der $\alpha,\alpha$-Diphenylhydroxymethylrest in 2- oder 3-Stellung des Chinolizidinkerns gebunden ist, wenn A die Phenylgruppe und n 4 ist, sowie ihre Säureadditionssalze und quaternären Salze.

2. Verfahren zur Herstellung der Verbindungen und ihrer Salze gemäss Anspruch 1, dadurch gekennzeichnet, dass man eine Verbindung der Formeln (II) oder (III):

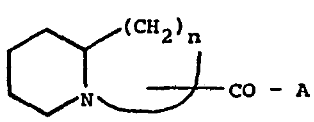
(II)

oder

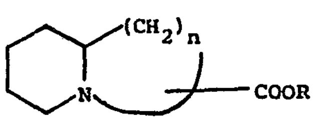
(III)

worin n und A die in Anspruch 1 angegebenen Bedeutungen haben, und R eine niedrige Alkylgruppe bedeutet, mit einer Metallverbindung der Formeln (IV) oder (V):

$$A — MgX \qquad (IV)$$

oder

$$A — Li \qquad (V)$$

worin A die oben angegebenen Bedeutungen hat und X ein Halogenatom bedeutet, umsetzt und ggf. die erhaltene Verbindung mit einer pharmazeutisch verträglichen, anorganischen oder organischen Säure oder mit einem Quaternisierungsmittel umsetzt.

3. Anticholinergische, antihistaminische, antiemetische und Antigeschwürmittel, dadurch gekennzeichnet, dass sie als Wirkstoff mindestens eine Verbindung der Formel (I), ihre Säureadditionssalze oder quaternären Salze enthalten.

**Revendications**

1. Dérivés $\alpha,\alpha$-disubstitués de la quinolizidine-méthanol et de l'indolizidine-méthanol de formule (I):

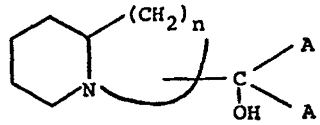

(I)

dans laquelle A est le groupe phényle ou 2-thiényle, et n est un nombre entier de 3 ou 4, où le reste $\alpha,\alpha$-diphényl-hydroxyméthyle est lié sur les positions 2 ou 3 du noyau quinolizidine quand A est le groupe phényle et que n vaut 4, ainsi que les sels acides d'addition et les sels quaternaires de ces dérivés.

2. Procédé pour la préparation des composés et de leurs sels selon la revendication 1, caractérisé par le fait qu'on fait réagir un composé de formule (II) ou (III):

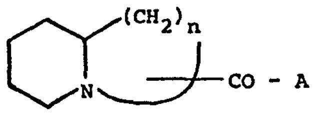

(II)

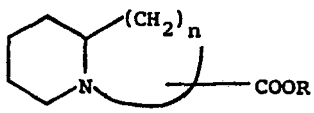

(III)

dans lesquelles n et A ont les significations mentionées dans la revendication (1), et R est un groupe alkyle inférieur, avec un composé métallique de formule (IV) ou (V).

A —MgX (IV)

ou

A — Li (V)

dans lesquelles A a les significations mentionnées ci-dessus, et X est un atome d'halogène, puis qu'éventuellement on fait réagir le composé obtenu avec un acide minéral ou organique, ou bien avec un agent de quaternisation pharmaceutiquement compatibles.

3. Agents anti-cholinergiques, anti-histaminiques, anti-émétiques et anti-tumeur, caractérisés par le fait qu'ils contiennent comme matière active au moins un composé de formule (I), ses sels acides d'addition ou ses sels quaternaires.

**Claims**

1. $\alpha,\alpha$-disubstituted quinolidine- and indolizidine methanol derivates with the formula (I):

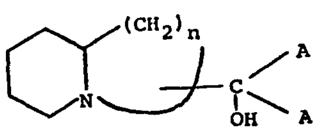

(I)

where A is the phenyl- or 2-thienyl group and n is a full number of 3 or 4, whereby the $\alpha,\alpha$-diphenyl hydroxymethyl residue is linked in the 2- or 3-position of the quinolizidine core, where A is the phenyl group and n is 4, as well as their acid addition salts and quaternary salts.

2. Process according to claim 1 for the preparation of said compounds and their salts, characterized in that: a compound of the formulae (II) and (III)

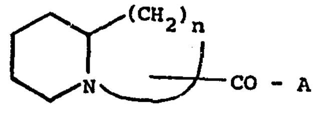

(II)

or

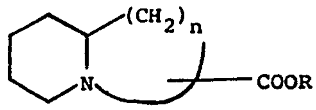

(III)

wherein n and A have the meanings given in claim 1 and where R is a low alkyl group, is reacted with a metal compound of the formulae (IV) and (V):

A — MgX (IV)

or

A — Li (V)

where A has the meanings given above and X is a halogen atom, and optionally the compound thus obtained is reacted with a pharmaceutically compatible anorganic or organic acid or with a quaternization agent.

3. Anticholinergic, antihistamine, antiemetic and antiulcer agent, characterized in that: it contains as the active component at least a compound of formula (I), its acid addition salts or its quaternary salts.